# EUROPEAN PATENT SPECIFICATION

(11) **EP 3 520 979 B1**
(45) Date of publication and mention of the grant of the patent: **28.12.2022**
(21) Application number: 18154761.3
(22) Date of filing: 01.02.2018
(51) Int. Cl.: B29C 43/00, B29C 43/22, A61M 37/00, B29C 59/00, B29C 59/02, B29C 43/48, B29K 33/00, B29K 69/00, B29L 31/00

(54) **MICRONEEDLE ARRAY AND METHOD OF MANUFACTURING A MICRONEEDLE ARRAY**
MIKRONADELANORDNUNG SOWIE VERFAHREN ZUR HERSTELLUNG VON EINER MIKRONADELANORDNUNG
RÉSEAU DE MICRO-AIGUILLES ET PROCÉDÉ DE FABRICATION D'UN RÉSEAU DE MICRO-AIGUILLES

(43) Date of publication of application: 07.08.2019
(73) Proprietor: SABIC Global Technologies B.V., 4612 PX Bergen op Zoom (NL)
(72) Inventor: VAN DE GRAMPEL, Robert, 4612 PX Bergen op Zoom (NL); DE BROUWER, Johannes, 4612 PX Bergen op Zoom (NL); JUSTER, Herwig, 4612 PX Bergen op Zoom (NL)
(74) Representative: Sabic Intellectual Property Group

(56) References cited:
- EP-A1- 3 006 078
- WO-A1-2019/082099
- US-A1- 2009 234 301
- US-A1- 2015 306 363
- US-A1- 2015 352 777

## Description

### FIELD OF INVENTION

The present disclosure relates generally to microneedle arrays, and to methods for producing and using microneedles, such as microneedles formed from a multilayer polymer using an embossing technique.

### BACKGROUND

Health and wellness monitoring of a patient often requires sampling fluid (e.g., blood or interstitial fluid). For example, individuals with diabetes often use a lancing device to puncture tissue and draw blood provided to a test device, such as a blood glucose meter. As another example, a syringe is used to acquire fluid from a patient. Depending on an individual's health, multiple fluid samples may be acquired per day and, in severe situations, continuous sampling and monitoring may be warranted. Frequent or continuous sampling and monitoring is often painful, uncomfortable (e.g., causes skin sensitivity), and inconvenient.

A microneedle array has potential to provide a pain-free or reduced-pain alternative to a syringe for sampling fluid from a patient for testing (e.g., blood glucose level, insulin level, medication level, etc.). Microneedles can be virtually painless because they do not penetrate deep enough to contact nerves and are typically limited to penetration of the outermost layer of the skin, unlike traditional syringes and hypodermic needles. Additionally, a less shallow penetration may also reduce the risk of infection or injury. Microneedles may also facilitate delivery of a more precise dosage of a therapeutic which enables the use of lower doses in treatments. Other advantages of microneedles for drug delivery include the simplified logistics (absence of required cold chain), ability for patient self-administration (no need for doctor, nurse, reduction of people transport). As used in this disclosure, a "hollow" microneedle is a microneedle with an internal channel extending through at least a portion of the length of the microneedle and opening through at least one outer surface of the microneedle to permit fluid communication through the microneedle. In contrast, a "solid" microneedle is a microneedle that does not include a channel extending through an exterior surface of the microneedle, such that fluid communication is not permitted through the microneedle.

Manufacturing of microneedle arrays has met many challenges to produce microneedle arrays at sufficient scale and efficiency such that they are cost effective. For example, given the relatively small sizes of microneedles and features of the microneedles, expensive production processes are needed. To illustrate, to have proper transcription of mold texture and shape to the molded part of a microneedle array, high flow may be necessary, especially having low viscosity at extremely high shear rates. Furthermore, good release from the production mold is important to reduce cycle time to improve the cost efficiency. The needles formed therefrom should exhibit good strength to prevent breaking of the microneedle during usage. Additionally, microneedles need to have a combination of properties for different phases of use. For example, microneedles need to have an aspect ratio and overall dimensions to obtain a sharp tip and a sharp blade to cut the skin, and length to penetrate the skin sufficiently. As another example, microneedles need to have mechanical and chemical properties to be able to withstand manufacturing and handling processes, tolerate (without degradation) interactions with therapeutic drugs, and functionally need to penetrate tissue (tip and cutting surface), remain in tissue, removed from tissue, and be handled before and after insertion, all without breaking.

While there are a number of benefits to the use of microneedles and considerations with respect to forming them, certain challenges remain in microneedle production. To illustrate such challenges, a conventional approach to form microneedle arrays (i.e., microneedles) includes injection molding a material, such as polycarbonate (PC), into a microneedle mold. Although PC has good overall stiffness for microneedle applications, microneedle arrays formed of PC (i.e., having tips of PC) using injection molding may tend to fail in filling the high aspect ratio of the microneedle molds (or the microneedle master structure) during processing, thereby resulting in poor replication of the needle tip. Thus, manufacturing of microneedle arrays faces challenges of identifying one or more techniques and/or one or more materials to manufacture microneedles that have certain aspect ratios for a sharp tip (e.g., good tip replication during manufacturing) and that have a combination of properties (e.g., mechanical and/or chemical) for different phases of microneedle use.

US 2009/0234301 A1 describes a microneedle array and a method for producing a microneedle array. EP 3 006 078 A1 describes a needle body manufacturing method and manufacturing device. US 2015/0352777 A1 describes the high-throughput manufacturing of microneedles. US 2015/0306363 A1 describes an article with hollow microneedles and a method of making the article. WO2019/082099 discloses a method of manufacturing a microneedle array.

### SUMMARY

The present disclosure describes a microneedle array, methods and systems for producing the microneedle array, and systems and methods for forming the present multilayer sheet from which the microneedle array is formed. As described herein, the microneedle array may be formed by hot embossing a co-extruded multilayer film (e.g., a first layer of polymethylmethacrylate (PMMA) and a second layer of PC). The hot embossing technique may advantageously enable a material (e.g., PMMA) to fill a mold cavity having a high aspect ratio thereby enabling good replication of the needle tip. Additionally, or alternatively, multilayer film formed by co-extrusion enables control of layering and layer thicknesses specific for use in formation of microneedle arrays. As an example, the multilayer film may have a first layer (e.g., a top layer) of PMMA and a second layer (e.g. a bottom layer) of PC. PMMA may function as a surface layer (e.g., a cap or top layer) of microneedle array and may provide acceptable microneedle geometric replication accuracy (e.g., tip sharpness and aspect ratio), provide resistance (e.g., does not degrade) to handling and environmental conditions, and provide sufficient hardness for microneedle use. PC can also provide toughness and durability to the microneedles and enable an appropriate amount of bending with permanent deformation of the needles limited and without breakage of the microneedles. Microneedle arrays formed from the multilayer sheet (having a first PMMA layer and a second PC layer) advantageously have aspect ratios for microneedles with sharp tips (of PMMA) to penetrate dermal surfaces (while still maintaining the benefit of being relatively pain free) and to maintain their structural integrity and strength during use. Accordingly, the present disclosure overcomes the existing challenges of forming microneedles with both high aspect ratios for a sharp tip (e.g., good tip replication during manufacturing) and a combination of properties (e.g., mechanical and/or chemical) for different phases of microneedle use, by hot embossing the co-extruded multilayer film of PMMA and PC.

According to aspects of the present disclosure, a method of forming a microneedle array may include forming a multilayer sheet of at least two layers, a first layer of polymethylmethacrylate (PMMA) and a second layer of PC. For example, the present disclosure describes a multilayer sheet, such as a co-extruded multilayer film, including a first layer (e.g., a top layer) of PMMA and a second layer (e.g. a bottom layer) of PC. Microneedle arrays of the present disclosure formed from the multilayer sheet (having a first PMMA layer and a second PC layer) have aspect ratios for microneedles with sharp tips (of PMMA) to penetrate dermal surfaces (while still maintaining the benefit of being relatively pain free) and to maintain their structural integrity and strength during use. For example, the microneedle array is formed from the multilayer sheet using a process such that the first layer of PMMA (of the multilayer sheet) forms a plurality of stiff microneedles (e.g., spikes) of the microneedle array and the second layer of PC (of the multilayer sheet) provides toughness for the microneedle array. As described herein, PMMA may function as a surface layer (e.g., a cap or top layer) and may provide acceptable microneedle geometric replication accuracy (e.g., tip sharpness and aspect ratio) for an embossing technique.

In various aspects, the microneedle array described herein exhibit both hardness and toughness that may be lacking in conventional microneedle arrays. For example, the combination of PMMA and PC in the microneedle array obtains the hardness of PMMA (especially in the tip area) and incorporates the toughness behavior of PC to form the microneedle array. Additionally, or alternatively, PMMA as the top layer of microneedle array provides resistance (e.g., does not degrade) to handling and environmental conditions, and provides sufficient hardness for microneedle use (e.g., being inserted into the biological barrier, remaining in place for up to a number of days, and being removed) while also enabling an appropriate amount of bending without breakage of the microneedles and with restriction of permanent deformation of the needles restricted limited. Additionally, in some implementations, the microneedle arrays possess a chemical resistance that fulfill regulatory critical to quality (CTQ) requirements, such that there is minimal or no chemical reaction among an active ingredient of a therapeutic, a carrier/coating, and the PMMA forming the microneedle structures during production, sterilization, storage, and/or during the use of the microneedle array.

The present disclosure also includes systems and methods for manufacturing the present microneedle arrays having the desired varying aspect ratio, strength, and mechanical performance sufficient to provide a sharp tip among the microneedles and a sharp blade to properly penetrate or cut the skin.. For example, the microneedle arrays can be manufactured using an embossing process (e.g., plate-to-plate, roll-to-plate, or roll-to-roll hot embossing process). In a hot embossing process, micro textures (of a mold) are imprinted on a polymer, such as a first PMMA layer of the multilayer sheet (including the first PMMA layer and a second PC layer). Accordingly, in such hot embossing methods, a method includes heating (during a hot embossing process) the multilayer sheet and pressing the multilayer sheet between a tool (e.g., a mold) and a roller to displace a portion of the multilayer sheet into a plurality of recesses of the mold to form a plurality of microneedle structures on the first layer of PMMA. As a result, hot embossing may be used as a cost effective (e.g., low cost) process for mass production and large area texturing of geometrically accurate, defect free microneedle structures (e.g., a microneedle array). Additionally or alternatively, systems and methods described herein allow arrays (e.g., large arrays) of microneedles to be manufactured more rapidly than prior art methods. As another result, microneedle arrays formed with the present systems and methods need not be singular, discrete arrays, but can instead include one or more arrays extending along an elongated multilayer sheet of polymer. For example, a multilayer sheet of polymer with a length that is five or more times its width can have multiple microneedle arrays extending along a majority of its length.

The invention provides a microneedle array comprising: a multilayer polymer having a first side and an opposing second side, the multilayer polymer defining a plurality of microneedles on the first side, each microneedle having a distal end and a base between the distal end and the second side of the multilayer polymer, the base of each of the microneedles having a cross-sectional area larger than that of the respective distal end; where the multilayer polymer comprises: a first layer having the first side and that includes polymethylmethacrylate (PMMA); and a second layer having the second side and that includes polycarbonate (PC). In accordance with the invention a hardness of the first layer comprises a pencil hardness of H or greater.

In some of the foregoing embodiments of the present apparatuses, the multilayer polymer includes fewer than three layers. In some such embodiments, the first layer is in contact with the second layer.

In some of the foregoing embodiments of the present apparatuses, the multilayer polymer includes three or more layers. In some such embodiments, the multilayer polymer further comprises at least a third layer positioned between the first layer and the second layer. In some such embodiments, the third layer includes polyester or a resin. Additionally, or alternatively, in some embodiments, the third layer includes a polymer selected from the group consisting of polycarbonate (PC), polymethylmethacrylate (PMMA), liquid-crystal polymer (LCP), polyether ether ketone (PEEK), fluorinated ethylene propylene (FEP), polysulfone (PSU), polyethylenimine, polyetherimide, polyimide (PI), polycarbonate copolymer (PC COPO), cyclic olefin copolymer (COC), cyclo olefin polymer (COP), polyamide (PA), acrylonitrile butadiene styrene (ABS), and polyphenylene ether (PPE).

In some of the foregoing embodiments of the present apparatuses, a surface of the first layer that corresponds to the first side is an exposed surface of the plurality of microneedles. In some such embodiments, the first layer comprises a cross-linked material that includes the PMMA. Additionally, or alternatively, in some embodiments, a hardcoat layer is coupled to the first side of the multilayer polymer.

The invention also provides a method of manufacturing a microneedle array, the method comprising: disposing a multilayer polymer between a pressing device and a surface of a tool with a temperature of at least a portion of the multilayer polymer in contact with the tool above a glass transition temperature of the multilayer polymer, the tool defining a plurality of cavities, each of the cavities extending from a base at the surface to a distal end within the tool to define a negative mold of a microneedle, the base of each of the cavities having a cross-sectional area larger than that of the respective distal end; and pressurizing at least a portion of the multilayer polymer between the pressing device and the tool such that the multilayer polymer: defines a layer of polymer between the pressing device and the surface of the tool, the multilayer polymer having a first side facing the tool and a second side facing the pressing device; and flows into the cavities until the polymer substantially fills the cavities to form a plurality of microneedles on the first side; where the multilayer polymer comprises: a first layer having the first side and that includes polymethylmethacrylate (PMMA); and a second layer having the second side and that includes polycarbonate (PC). In some such embodiments, the first layer has a first glass transition temperature, the second layer has a second glass transition temperature, and the glass transition temperature of the multilayer polymer comprises the lower of the first glass transition temperature and the second glass transition temperature.

In some of the foregoing embodiments of the present methods, the present methods further comprise forming the multilayer polymer. In some such embodiments, forming the multilayer polymer comprises: receiving a first material comprising the PMMA; forming the first layer; receiving a second material comprising the PC; forming the second layer; and coupling the first layer and the second layer to form the multilayer polymer. In a particular embodiment, the first layer is formed by a first extrusion process and the second layer is formed by a second extrusion process.

The invention also provides a microneedle array formed by any of the foregoing embodiments of the present methods.

Related to the invention is a system (e.g., for manufacturing a microneedle array from one or more sheets of polymer) comprising: a frame; a tool rotatably coupled to the frame around a rotational axis, the tool having a surface and defining a plurality of cavities, each of the cavities extending from a base at the surface to a distal end within the tool to define a negative mold of a microneedle, the base of each of the cavities having a cross-sectional area larger than that of the respective distal end; and a first roller disposed at a first angular position relative to the rotational axis of the tool, the first roller having a radial surface configured to press a multilayer polymer against the surface of the tool to cause the multilayer polymer to flow into the cavities in the surface of the tool to define a plurality of microneedles on a first side of the multilayer polymer, each microneedle having a distal end and a base between the distal end and the second side of the multilayer polymer, the base of each of the microneedles having a cross-sectional area larger than that of the respective distal end; and where the multilayer polymer comprises: a first layer having the first side and that includes polymethylmethacrylate (PMMA); and a second layer having the second side and that includes polycarbonate (PC).

In some of the foregoing embodiments of the present systems, the present systems further comprise: a first extrusion device configured to form the first layer; and a second extrusion device configured to form the second layer.

In some of the foregoing embodiments of the present systems, the present systems further comprise: a dispenser spindle coupled to the frame and configured to rotatably support a dispenser roll of a sheet of the multilayer polymer; and a receiver spindle coupled to the frame and configured to rotatably support a receiver roll to receive portions of the sheet of polymer having microneedles after definition of the microneedles on a first side of the multilayer polymer.

As used herein, an ordinal term (e.g., "first," "second," "third," etc.) used to modify an element, such as a structure, a component, an operation, etc., does not by itself indicate any priority or order of the element with respect to another element, but rather merely distinguishes the element from another element having a same name (but for use of the ordinal term). The term "coupled" is defined as connected, although not necessarily directly, and not necessarily mechanically; two items that are "coupled" may be unitary with each other. The terms "a" and "an" are defined as one or more unless this disclosure explicitly requires otherwise. The term "substantially" is defined as largely but not necessarily wholly what is specified (and includes what is specified; e.g., substantially 90 degrees includes 90 degrees and substantially parallel includes parallel), as understood by a person of ordinary skill in the art. In any disclosed embodiment, the term "substantially" may be substituted with "within [a percentage] of' what is specified, where the percentage includes .1, 1, or 5 percent; and the term "approximately" may be substituted with "within 10 percent of' what is specified. The phrase "and/or" means and or or. To illustrate, A, B, and/or C includes: A alone, B alone, C alone, a combination of A and B, a combination of A and C, a combination of B and C, or a combination of A, B, and C. In other words, "and/or" operates as an inclusive or.

The terms "comprise" (and any form of comprise, such as "comprises" and "comprising"), "have" (and any form of have, such as "has" and "having"), and "include" (and any form of include, such as "includes" and "including"). As a result, an apparatus that "comprises," "has," or "includes" one or more elements possesses those one or more elements, but is not limited to possessing only those one or more elements. Likewise, a method that "comprises," "has," or "includes" one or more steps possesses those one or more steps, but is not limited to possessing only those one or more steps.

Any embodiment of any of the systems, methods, and article of manufacture can consist of or consist essentially of - rather than comprise/have/include - any of the described steps, elements, and/or features. Thus, in any of the claims, the term "consisting of' or "consisting essentially of' can be substituted for any of the open-ended linking verbs recited above, in order to change the scope of a given claim from what it would otherwise be using the open-ended linking verb. Additionally, the term "wherein" may be used interchangeably with "where."

Some details associated with the embodiments are described above, and others are described below. Other implementations, advantages, and features of the present disclosure will become apparent after review of the entire application, including the following sections: Brief Description of the Drawings, Detailed Description, and the Claims.

### BRIEF DESCRIPTION OF THE DRAWINGS

The following drawings illustrate by way of example. For the sake of brevity and clarity, every feature of a given structure is not always labeled in every figure in which that structure appears. Identical reference numbers do not necessarily indicate an identical structure. Rather, the same reference number may be used to indicate a similar feature or a feature with similar functionality, as may non-identical reference numbers. The figures are drawn to scale (unless otherwise noted), meaning the sizes of the depicted elements are accurate relative to each other for at least the embodiment depicted in the figures. Views identified as schematics are not drawn to scale.
**FIG. 1A** is a diagram that that illustrates an example of a multilayer sheet.
**FIG. 1B** is a diagram that that illustrates a cross-section view of an example of a microneedle array formed from the multilayer sheet of FIG. 1A.
**FIGS. 2A and 2B** are perspective and side views, respectively, of an example of a microneedle included in the microneedle array of FIG. 1B.
**FIG. 3** is a diagram that illustrates examples of microneedle arrays.
**FIG. 4** is a cross-section view of another example of a microneedle array formed from a multilayer sheet.
**FIG. 5** is a perspective view of an example of a system for producing a multilayer sheet.
**FIG. 6A** is a diagram that illustrates an example a cross-sectional view of a set of molds for forming a microneedle array.
**FIG. 6B** is a diagram that illustrates an example of stages of an embossing process to form a microneedle array using the set of molds of FIG. 6A.
**FIG. 7** is a schematic view of an example of a system for manufacturing a microneedle array.
**FIG. 8** is a flowchart illustrating an example of a method of manufacturing a microneedle array.

### DETAILED DESCRIPTION OF ILLUSTRATIVE EMBODIMENTS

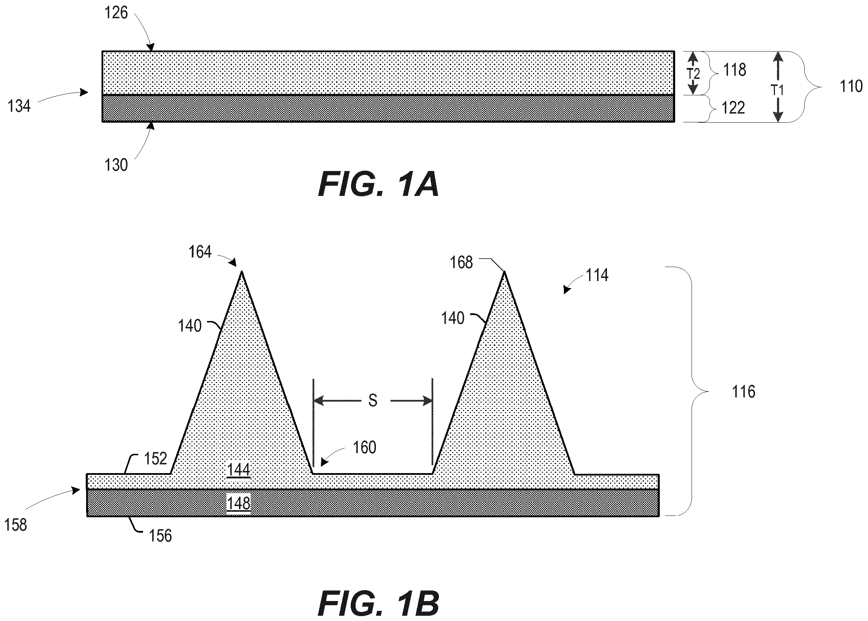

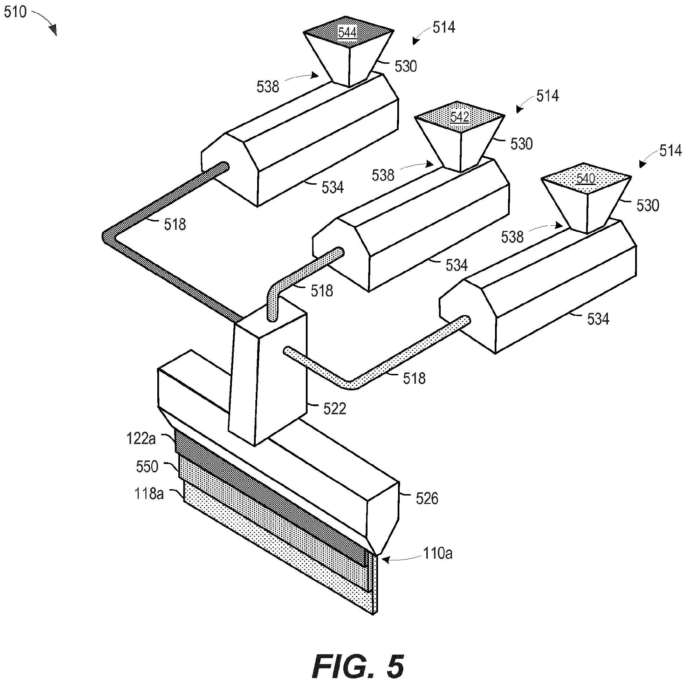

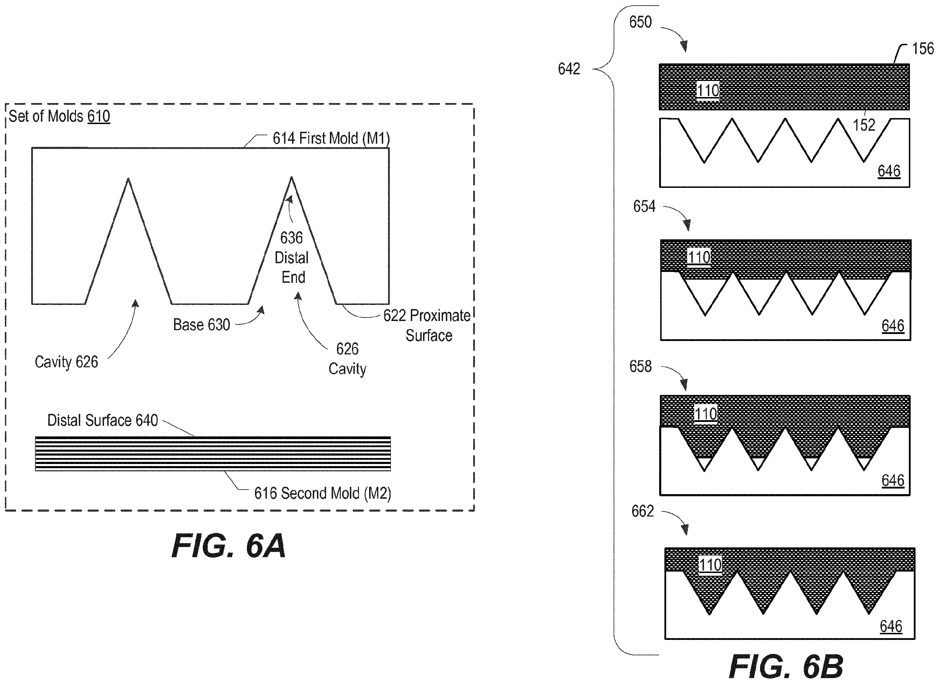

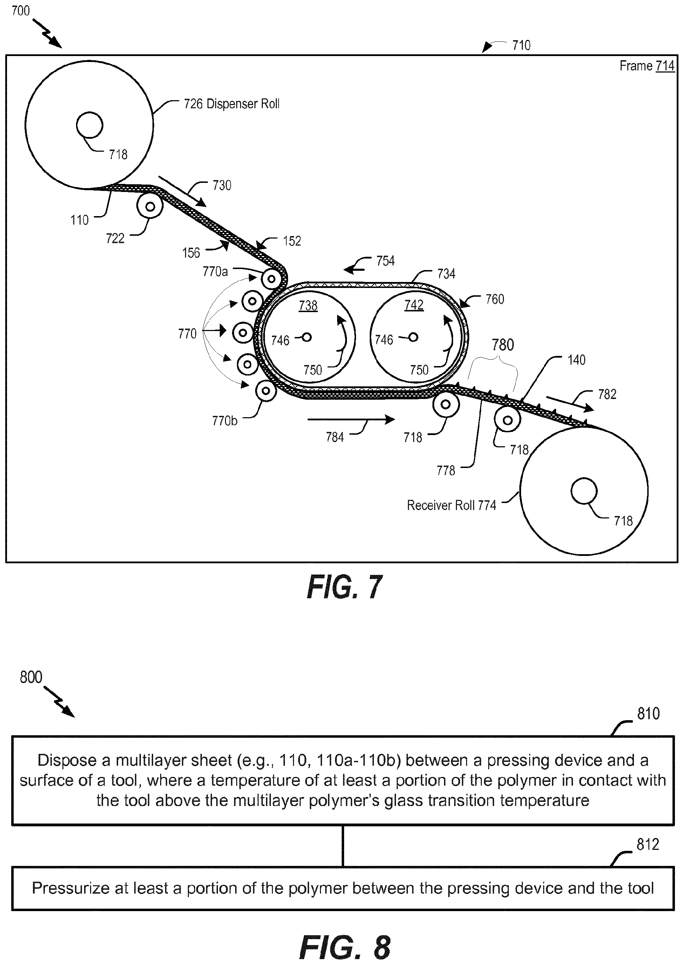

Referring to FIGS. 1A-B, FIG. 1A shows a cross-sectional view of a multilayer sheet 110 (e.g., a multilayer polymer) for use in forming a microneedle array 114 and FIG. 1B shows a cross-sectional view of microneedle array 114 (formed from multilayer sheet 110). Multilayer sheet 110 may be formed via an extrusion technique, as described herein with reference to FIG. 5 Additionally or alternatively, multilayer sheet 110 may be used in an embossing process, such as a hot embossing process, as described herein with reference to FIGS. 6B and 7.

Referring to FIG. 1A, multilayer sheet 110 includes two or more layers, such as a first layer 118 and a second layer 122. Multilayer sheet 110 includes a first surface 126 (corresponding to a surface of first layer 118) and a second surface 130 (corresponding to a surface of second layer 130). As shown, first layer 118 is coupled to (e.g., is in contact with) second layer 122 via an interface 134 that is intermediate surfaces 126, 130. A thickness T1 (e.g., from surface 126 to surface 130 measured in a direction that substantially normal to surfaces 126, 130) of sheet 110 is from 300 micrometers (µm) to 2,500 µm (e.g., from 500 µm to 2,000 µm). In other implementations, thickness T1 of sheet 110 is less than 300 µm or is greater than 2,500 µm.

First layer 118 includes a first polymer (i.e., PMMA) and second layer 122 includes a second polymer (e.g., PC). In some implementations, first layer 118 includes PMMA and does not include PC. Additionally, or alternatively, second layer 122 include PC and does not include PMMA. In a particular implementation, multilayer sheet 110 includes a LEXAN OQ6DA film available from SABIC^{™}.

Thicknesses of individual layers of the multilayer sheet 110 can be selected to achieve desired properties and/or functions of the multilayer sheet 110 and/or of the microneedle array 114, as described further herein. For example, a thickness T2 of first layer 118 can be greater than or equal to 25 µm. In other implementations, thickness T2 of first layer 118 can be less than 25 µm. In some implementations, thickness T2 of first layer 118 can be up to 40% of thickness T1 of sheet 110 (as described above). To illustrate, if thickness T1 of sheet 110 is 2,000 µm, thickness T2 of first layer 118 can be within a range of 0 to 800 µm (e.g., from 25 µm to 800 µm).

PMMA has positive characteristics for microneedle applications, such as processing capabilities (e.g., can be processed over a range of processing parameters), ultraviolet radiation (UV) stability, optical properties (e.g., gloss, transparency, clarity, haze, color, surface aspect, and/or refractive index), and is Bisphenol A (BPA) free. PMMA can also be formed, using an embossing technique, into structures that have a hardness greater than or equal to a pencil hardness of H.

Polycarbonate (PC) may be used to provide toughness for microneedle applications. The term "polycarbonate" (or "polycarbonates"), as used herein, includes copolycarbonates, homopolycarbonates and (co)polyester carbonates. The term polycarbonate can be further defined as compositions have repeating structural units of the formula (1): 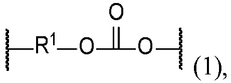 in which at least 60 percent of the total number of R1 groups are aromatic organic radicals and the balance thereof are aliphatic, alicyclic, or aromatic radicals. In a further aspect, each R1 is an aromatic organic radical and, more preferably, a radical of the formula (2):

-A1-Y1-A2- (2),

where each of A1 and A2 is a monocyclic divalent aryl radical and Y1 is a bridging radical having one or two atoms that separate A1 from A2. In various aspects, one atom separates A1 from A2. For example, radicals of this type include radicals such as -O-, -S-, -S(O)-, -S(O2)-, -C(O)-, methylene, cyclohexyl-methylene, 2-[2.2.1]-bicycloheptylidene, ethylidene, isopropylidene, neopentylidene, cyclohexylidene, cyclopentadecylidene, cyclododecylidene, and adamantylidene. The bridging radical Y1 may be a hydrocarbon group or a saturated hydrocarbon group, such as methylene, cyclohexylidene, or isopropylidene, as illustrative examples.

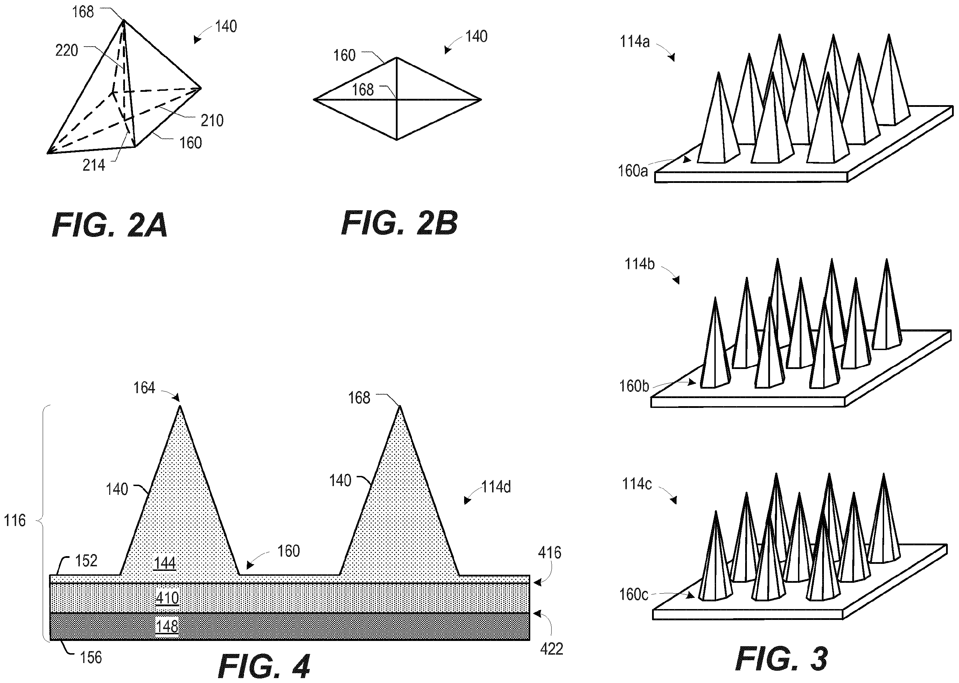

In some implementations, multilayer sheet 110 may include one or more additional layers positioned between first and second layers 118, 122, as described with reference to at least FIGS. 4 and 5. The one or more additional layers may be used to provide durability, flexibility, stiffness, and/or toughness to array 114 formed from the multilayer sheet. In a particular implementation, multilayer sheet 110 includes alternating layers of PMMA and PC such that a top layer (having surface 126) includes PMMA and a bottom layer (having surface 130) includes PC. In such implementations, each layer of PMMA can have a corresponding thickness (as described above).

In some implementations, multilayer sheet 110 may be formed with one or more protective skin layers, such as a first skin layer coupled to (e.g., in contact with) first surface 126, a second skin layer coupled to (e.g., in contact with) second surface 130, or both. These skin layers may be extruded with the multilayer sheet, as described with reference to FIG. 5, such that the first skin layer is extruded on first layer 118, second skin layer is extruded on second layer 130, or both. The protective skin layer(s) may prevent physical damage to multilayer sheet 110 during cooling after extrusion, during storage, or other manipulation. For example, the formed multilayer sheet 110 (including the one or more skin layers) may be stored on a roll prior to a molding process and at least one of the one or more skin layers may be removed prior to the molding process being performed. The one or more protective skin layers may include polypropylene or polycarbonate, as illustrative examples.

First layer 118, a second layer 122, or both, may include various additives ordinarily incorporated into polymer compositions, with the proviso that the additive(s) are selected so as to not significantly adversely affect the desired properties of the polymer of the layer (e.g., the additives have good compatibility with the polymer). Such additives can be mixed at a suitable time during the mixing of the components for forming the material comprising the multilayer sheet. An exemplary polymer (of one of the layers of multilayer sheet 110 of the present disclosure) may include additives, such as a mold release agent to facilitate ejection of a formed microneedle array from the mold assembly. Examples of mold release agents include both aliphatic and aromatic carboxylic acids and their alkyl esters, such as stearic acid, behenic acid, pentaerythritol tetrastearate, glycerin tristearate, and ethylene glycol distearate, as illustrative examples. Mold release agents can also include polyolefins, such as high-density polyethylene, linear low-density polyethylene, low-density polyethylene, and similar polyolefin homopolymers and copolymers. Additionally, some compositions of mold release agents may use pentaerythritol tetrastearate, glycerol monostearate, a wax, or a poly alpha olefin. Mold release agents are typically present in the composition at 0.05 to 10 wt %, based on total weight of the composition, such as 0.1 to 5 wt %, 0.1 to 1 wt%, or 0.1 to 0.5 wt%. Some mold release agents 1 have high molecular weight, typically greater than or equal to 300, to prohibit loss of the release agent from the molten polymer mixture during melt processing.

In addition to including a corresponding polymer and/or a mold release agent, first layer 118, second layer 122, or both, may further include one or more additives intended to impart certain characteristics to microneedle array 114 formed by a mold assembly, as described herein. As illustrative examples, the polymer (of first layer 118, second layer 122, or both) may include an impact modifier, flow modifier, antioxidant, heat stabilizer, light stabilizer, ultraviolet (UV) light stabilizer, UV absorbing additive, plasticizer, lubricant, antistatic agent, antimicrobial agent, colorant (e.g., a dye or pigment), surface effect additive, radiation stabilizer, or a combination thereof. To illustrate, in a particular implementation, the first layer 118 can include a heat stabilizer and an ultraviolet light stabilizer.

Additionally, or alternatively, first layer 118, second layer 122, or both, may exhibit excellent release, as measured by ejection force (N) and coefficient of friction. In some implementations, first layer 118, second layer 122, or both, include (i) high flow at high shear conditions to allow good transcription of mold texture and excellent filling of the finest mold features, (ii) good strength and impact (as indicated by ductile Izod Notched Impact at room temperature and modulus), and/or (iii) high release to have efficient de-molding and reduced cooling and cycle time during molding. Microneedle arrays 114 formed from sheet 110 may have sufficient mechanical strength to remain intact (i) while being inserted into the biological barrier, (ii) while remaining in place for up to a number of days, and (iii) while being removed.

Referring to FIG. 1B, microneedle array 114 (formed from multilayer sheet 110) includes a plurality of microneedles 140. Array 114 includes a multilayer polymer structure 116 having a first layer 144 and a second layer 148. Multilayer polymer structure 116 corresponds to (e.g., is formed from) multilayer sheet 110 such that first layer 144 corresponds to (e.g., is formed form) first layer 118 of sheet 110 and includes PMMA, and second layer 148 corresponds to (e.g., is formed form) second layer 122 of sheet 110 and includes PC. In a particular implementation, the first layer 144 includes a cross-linked material that includes the PMMA. As shown, an interface 158 between layers 144, 148 is substantially planar. In other implementations, interface 158 may be irregular, such as planar, curved, or a combination thereof. For example, in a particular implementation, a portion of second layer 148 may extend into a base region of microneedle 140. It is noted that the position of interface 158 in FIG. 1B is for illustration purposes and, in other implementations, interface 158 may be positioned (e.g., located) closer to a first side 152 of array 114 or closer to a second side 156 of array 114. For example, the position of interface 158 may be determined based on thicknesses of one or more layers of multilayer sheet 110 (from which microneedle array 114 is formed), processing conditions (e.g., temperature, pressure, duration, etc.) of an array formation process, such as embossing, or a combination thereof. In such an implementation, each layer of PMMA (of sheet 110) may have the same thickness or at least one layer of PMMA (of 110) has a different thickness from the other layers of PMMA, and/or each layer of PC (of 110) may have the same thickness or at least one layer of PC (of 110) has a different thickness from the other layers of PC.

As shown, array 114 has a first side 152 (e.g., a first surface) and a second side 156 (e.g., a second surface), and microneedles 140 extend from first side 152. Although array 114 is illustrated as including two microneedles 140, in other implementations, microneedle array can include a single microneedle or more than two microneedles, such as tens, hundreds, or thousands of microneedles. In some implementations, array 114 can include a coating (e.g., a hardcoat layer) coupled to first side 152. For example, the coating may include a therapeutic drug (e.g., isosorbide) or other material.

Microneedle 140 includes a base 160 and a distal end 164. Base 160 of a particular microneedle has a cross-sectional area larger than that of the respective distal end 164 of the particular microneedle. A tip 168 is positioned at distal end 164 of microneedle 140. Sharpness of tip 168 may be expressed by a radius of curvature(s) of tip 168. In a particular implementation, sharpness of tip 168 is less than 10 µm. In another implementation, sharpness of tip 168 is less than 20 µm. Alternatively, sharpness of tip 168 may be greater than or equal to 20 µm. Although microneedle 140 is illustrated as having a single tip, in other implementations, microneedle 140 may include multiple tips, a blade, or a combination thereof.

Additional characteristics (e.g., dimensions) of microneedle 140 are described with reference to FIGS. 2A and 2B, which show perspective and side views, respectively, of microneedle 140. Referring to FIGS. 2A and 2B, microneedle 140 is shaped as a pyramid and has base 160 and tip 168. As shown, base 160 has a rhomboid cross-sectional shape with a primary maximum transverse dimension 210 and a secondary maximum transverse dimension 214 measured perpendicular to primary maximum transverse dimension 210. Maximum transverse dimension 210 is greater than or equal to a secondary maximum transverse dimension 214 and may range from 75 µm to 350 µm. In other implementations, maximum transverse dimension 210 may be less than or equal to 75 µm, or is greater than or equal to 350 µm.

Microneedle 140 also has a height 220 that is measured perpendicular to both of the primary and secondary maximum transverse dimensions (210 and 214) of base 160. Height 220 of microneedle 140 is from 200 µm to 1,100 µm (e.g., from 400 µm to 1,000 µm, or from 400 µm to 800 µm). In a particular implementation, height 220 is 250 µm. In other implementations, height 220 is less than 250 µm or is greater than 1,000 µm.

In some implementations, height 220 of microneedle 140 is from one (1) to five (5) times primary maximum transverse dimension 210 (e.g., a height 220 of 1,000 µm and a primary maximum transverse dimension 210 from 200 µm to 1,000 µm, inclusive of 200 µm and 1,000 µm). In some such implementations, height 220 is from two (2) to four (4) times primary maximum dimension 210 (e.g., a height of 1,000 µm and a primary maximum dimension 210 from 250 µm to 500 µm).

In some implementations, height 220 is from one (1) to five (5) times secondary maximum transverse dimension 214 (e.g., a height 220 of 1,000 µm and a secondary maximum transverse dimension 214 from 200 µm to 1,000 µm, inclusive of 200 µm and 1,000 µm). In some such implementations, height 220 is from two (2) to four (4) times secondary maximum dimension 214 (e.g., a height of 1,000 µm and a secondary maximum dimension 214 from 250 µm to 500 µm). In one particular example, primary maximum transverse dimension 210 is 170 µm, secondary maximum transverse dimension 214 is 120 µm, and height 220 is 250 µm.

As shown in FIGS. 2A and 2B, microneedle 140 has a rhomboid cross-sectional shaped base (e.g., 160) and has a pyramid shape defined by four planar surface. In other implementations, base 160 may have a different cross-sectional shape, such as ellipsoid, triangular, square, rectangular, hexagonal, octagonal, star, or other shape. For example, referring to FIG. 3 examples (114a-114c) of microneedle array 114 are shown in which a first microneedle array 114a includes a base 160a having a rectangular (e.g., square) cross-sectional shape, a second microneedle array 114b includes a base 160b having a hexagonal cross-sectional shape, and a third microneedle array 114c includes a base 160c having an octagonal cross-sectional shape. Additionally, or alternatively, in other implementations, microneedle 140 can have any suitable outer surface profile or shape. For example, outer surfaces may be curved or curvilinear (e.g., concave) to result in a relatively sharper tip 168, or one or more blades along the vertices along which the outer surfaces of the microneedle meet one another may be curved or curvilinear (e.g., concave). In yet further configurations, the present microneedles can have any suitable shape that permits the microneedle to puncture a patient's skin as contemplated by this disclosure. It is noted that, in some implementations, at least a portion of microneedles 140 may vary in size (e.g., primary maximum transverse dimension 210, secondary maximum transverse dimension, height 220, cross-sectional base shape, and/or profile) relative to each other. This variation in size creates a varying aspect ratio in the microneedle array 114.

Additionally, with reference to FIG. 1B, spacing or distance (S) between two adjacent or closest microneedles may be different than or the same as primary maximum transverse dimension 210. To illustrate, spacing (S) may be less than primary maximum transverse dimension 210. In a particular implementation, spacing (S) may be within a range from 75 µm to 2 millimeters (mm). In other implementations, spacing (S) is greater than 2 mm.

As shown in FIGS. 1B, 2A, and 2B, microneedle 140 is a "solid" microneedle that does not include a channel extending through the microneedle. In other implementations, microneedle 140 may be a "hollow" microneedle with an internal channel extending through at least a portion of the microneedle.

The shape of the present microneedles (e.g., 140) are not particularly limited, but certain considerations may guide selection and design of different shapes. For example, the shape of a mold cavity to form the present microneedles (e.g., 140) may impact the ability to manufacture molds. Additionally, the shape of the mold cavity may impact the ease with which a microneedle array (e.g., 110) can be separated from a tool or mold after the polymer solidifies (e.g., the ease or lack thereof with which the microneedles can be removed from the cavities). For example, draft angles in the mold greater than 0.5 degrees may facilitate removal of a molded microneedle array from a mold. Additionally, or alternatively, the shape of the microneedle can impact the ability of the microneedle to puncture a patient's skin. For example, a microneedle may be designed to be strong enough to pierce the patient's skin and, while a broader base may result in a stronger microneedle, the increased angles of the sides of such a microneedle (with a broader base) may cause relatively greater trauma to the patient's skin. Thus, the aspect ratios discussed in this disclosure may be selected to result in microneedles with tips that are sharp enough to puncture a patient's skin with relatively low force, while causing minimal disruption to the surface of the patient's skin (e.g., the stratum corneum).

In a particular implementation, microneedle array 114 includes a multilayer polymer (e.g., multilayer polymer structure 116) having first side 152, such as an exposed surface, and an opposing second side 156. The multilayer polymer defines a plurality of microneedles 140 on the first side 152. Each microneedle 140 having a distal end 164 and a base 160 between the distal end 164 and the second side 156. Base 160 of each of the microneedles 160 has a cross-sectional area larger than that of the respective distal end 164. Multilayer polymer includes a first layer 144 and a second layer 148. First layer 144 has the first side 152 and includes polymethylmethacrylate (PMMA). Second layer 148 has the second side 156 and includes polycarbonate (PC).

Additionally, forming microneedle array 114 may include forming a plurality of depressions at first surface 126 of multilayer sheet 110, as described further with reference to FIGS. 6B and 7. Formation of protrusions (e.g., microneedles 140) may occur by heating multilayer sheet 110 to a minimum of the glass transition temperature of the multilayer sheet 110 (e.g., at least to a minimum temperature of the glass transition temperature of first layer 118) and displacing at least a portion (e.g., at least a portion of first layer 118) of multilayer sheet 110 into a plurality of recesses, such as cavities of a mold, corresponding to a configuration for microneedle array 114. In a specific example, at least a portion of multilayer sheet 110 may be displaced into a plurality of recesses of a hot embossing mold.

As described with reference to FIGS. 1A and 1B, microneedle array 114 is formed from multilayer sheet 110 having first layer 118 of PMMA and second layer 122 of PC. The multilayer sheet 110 may advantageously be designed and constructed such that a relative ratio of layer thicknesses are selected to improve array formation processing (e.g., speed, robustness of the process, release from a mold, and/or accuracy of geometric replication) and to improve the final properties of the microneedle itself (e.g., mechanical, chemical, and/or optical properties). Multilayer sheet 110 may be hot embossed to form array 114 such that layer 118 of PMMA corresponds to side 152 (e.g., surface) that defines microneedles 140 (e.g., microneedles 140 include PMMA).

PMMA may function as a surface layer (e.g., a cap or top layer) of microneedle array 114 and may provide acceptable microneedle geometric replication accuracy (e.g., tip sharpness and aspect ratio) for one or more molding techniques (e.g., embossing). Additionally, or alternatively, PMMA as the top layer of microneedle array 114 provides resistance (e.g., does not degrade) to handling and environmental conditions, and provides sufficient hardness for microneedle use (e.g., while being inserted into the biological barrier, while remaining in place for up to a number of days, and while being removed), such that the microneedles enable an appropriate amount of bending with permanent deformation of the needles limited and without breakage of the microneedles. PC as second layer 148 (of array 114) may provide toughness to the microneedles 140. Thus, given layers 118, 122 of multilayer sheet 110 described herein to form array 114, microneedle array 114 formed according to the present disclosure (e.g., embossing) may have the benefit of a strong microneedle 140 (e.g., a top layer), such as PMMA, and a tough/durable base layer (e.g., a bottom layer), such as PC. Additionally, or alternatively, in some implementations, microneedles 140 formed according to the present disclosure may have the benefit of a strong tip, such as PMMA, and a tough/durable base, such as PC included in a base region of the microneedle array 114.

FIG. 4 shows another example 114d of the present microneedle array 114 of FIG. 1B. Relative to array 114 of FIG. 1, multilayer polymer structure 116 of microneedle array 114d includes a third layer 410 positioned between first layer 144 (e.g., PMMA) and second layer 148 (e.g., PC).

Third layer 410 includes a material selected to provide durability, flexibility, toughness, or a combination thereof, to array 114d formed from multilayer sheet 110 (having a third layer of material positioned between first layer 118 and second layer 122). These material may include a polyester, such as a semicrystalline polymer, polyethylene terephthalate (PET), polyethylene terephthalate glycol-modified (PETG), glycol-modified polycyclohexylenedimethylene terephthalate (PCTG), polycyclohexylenedimethylene terephthalate (PCT), isophthalic acid-modified polycyclohexylenedimethylene terephthalate (PCTA), and Tritan^{™} (a combination of dimethyl terephthalate, 1,4-cyclohexanedimethanol, and 2,2,4,4-tetramethyl-1,3-cyclobutanediol from Eastman Chemical). Additionally, or alternatively, the material may include a resin, such as Xylex^{™} (a combination of PC and an amorphous polyester), polybutylene terephthalate (PBT) (e.g., a resin from the Valox^{™} line of PBT), and/or a PET resin available from SABIC^{™}. Additionally, or alternatively, the material may include liquid-crystal polymer (LCP), polyether ether ketone (PEEK), fluorinated ethylene propylene (FEP), polysulfone (PSU), polyethylenimine, polyetherimide, polyimide (PI), polycarbonate (PC), polycarbonate copolymer (PC COPO), cyclic olefin copolymer (COC), cyclo olefin polymer (COP), polyamide (PA), acrylonitrile butadiene styrene (ABS), polyphenylene ether (PPE), or a combination thereof.

As shown, each of a first interface 416 (between layers 144, 410) and a second interface 422 (between layers 148, 410) is substantially planar. In other implementations, first interface 416 and/or second interface 422 may be planar, curved, or a combination thereof. For example, in a particular implementation, first interface 416 may be curved such that a portion of third layer 410 extends into a base region of microneedle 140 and/or includes a portion of side 152 (e.g., surface), and second interface 422 may be substantially planar.

Although illustrated as including a single layer between layers 144, 148, in some implementations, multiple layers that each include a corresponding material may be positioned (e.g., stacked or layered) between layers 144, 148. In such implementations, array 114d (and accordingly sheet 110 from which it is formed) may have seven or fewer layers positioned between. For example, third layer 410 may include one layer (in contact with first layer 144) having PBT and another layer (in contact with second layer 148) having PETG. In a particular implementation, multilayer polymer structure 116 includes three or more layers. Alternatively, in implementations where multiple layers are positioned between layers 144 and 148, array 114d (and accordingly sheet 110) may have more than seven layers. For example, in a particular implementation, array 114 may include four layers that alternate between PMMA and PC such that a top layer (having side 152) includes PMMA and a bottom layer (having side 156) includes PC.

Microneedle array 114d beneficially includes a strong top layer (including a strong tip), such as PMMA, and a tough bottom layer, such as PC, as described above with reference to array 114 of FIG. 1B. Additionally, array 114d advantageously includes third layer 410 that provides stiffness, flexibility, durability, and/or toughness to array 114d (e.g., microneedles 140).

Referring to FIG. 5, an example of a system 510 for producing a multilayer sheet 110a (e.g., sheet 110) is shown. System 510 is configured for use in an extrusion process to form sheet 110 or other examples (e.g., 110a) of multilayer sheet 110, as described herein.

System 510 includes extruders 514, feed tubes 518, feedblock 522, and die 526. As shown, system 510 includes three extrudes 514, however, in other implementations, system 510 can include two extruders 514 or more than three extruders 514.

Each extruder 514 includes a hopper 530 and barrel 534 coupled to the hopper 530, such as via a feed throat 538. Hopper 530 is configured to receive material (e.g., pellets, granules, flakes, and/or powders) that is provided (e.g., gravity fed or force fed) from the hopper 530 to barrel 534 via feed throat 538. As shown, a first hopper has received a first material 540 (e.g., PMMA), a second hopper has received a second material 542 (e.g., a material of third layer 410), and a third hopper has received a third material 544 (e.g., PC). In some implementations, another material can be combined with one or more of materials 540-544 provided to hoppers 530. The other material can include an impact modifier, flow modifier, antioxidant, heat stabilizer, light stabilizer, ultraviolet (UV) light stabilizer, UV absorbing additive, plasticizer, lubricant, antistatic agent, antimicrobial agent, colorant (e.g., a dye or pigment), surface effect additive, radiation stabilizer, or a combination thereof, as illustrative examples.

Each hopper 530 provides its material into its corresponding barrel 534. The material is gradually melted in barrel 534 by the mechanical energy (e.g., pressure) generated by turning screws, by heaters arranged along barrel 534, or both. Heated (e.g., molten or melted) material is provided from barrel 534 via feed tube 518 to feedblock 522 that combines received heated materials as stacked layers. From feedblock 522, the stacked layers of heated materials are provided to die 526, which outputs multilayer sheet 110a. As shown, multilayer sheet 110a includes first layer 118a (e.g. PMMA), a second layer 122a (e.g., PC), and a third layer 410 positioned between first and second layers 118a, 122a. In some implementations, third layer 550 is associated with (e.g., corresponds to) third layer 410 of array 114d (e.g., third layer 410 is formed from third layer 550). In other implementation, system 510 includes two extruders and extruder 514 with material 542 is omitted such that die outputs multilayer sheet 110 of FIG. 1A.

In some implementations, system 510 includes a layer multiplier positioned between feedblock 522 and die 526. The layer multiplier may include one or more multiplier elements that are each configured to increase (e.g., double) a number of received layers. For example, in an implementation where only two hoppers (e.g., a first hopper for PMMA and a second hopper for PC) are used in system 510, two layers (e.g., a first layer of PMMA and a second layer of PC) may be provided to a first multiplier element that outputs four layers including alternating layers of PMMA and PC (e.g., the first PMMA layer, the second PC layer, a third PMMA layer, and a fourth PC). If the four layers are provided to a second multiplier element, the second multiplier element would output eight alternating layers of PMMA and PC.

In a particular implementation, system 510 includes an even number hoppers (e.g., half of the hoppers having PMMA and the other half having PC) and die 526 is configured to output a multilayer sheet including alternating layers of PMMA and PC such that a top layer includes PMMA and a bottom layer (having surface 130) includes PC. In such an implementation, each layer of PMMA may have the same thickness or at least one layer of PMMA has a different thickness from the other layers of PMMA, and/or each layer of PC may have the same thickness or at least one layer of PC has a different thickness from the other layers of PC. As an illustrative example, the system 510 includes four hoppers and the multilayer sheet includes four alternating layers of PMMA and PC.

In some implementations, system 510 may be configured to produce multilayer sheet 110a that includes a first protective skin layer coupled to (e.g., in contact with) layer 118a, a second protective skin layer coupled to (e.g., in contact with) layer 122a, or both. The protective skin layer(s), such as polypropylene or polycarbonate, may prevent physical damage to multilayer sheet 110a during cooling after extrusion, during storage, or other manipulation. For example, the formed multilayer sheet may be stored on roll before a molding process (e.g., an embossing process). Removal of the skin layer may be achieved by physical removal, such as peeling.

As described with reference to FIG. 5, system 510 is configured to form a multilayer sheet (e.g., 110, 110a) having a layer of PMMA and a layer of PC. For example, in conjunction with feedblock 522 and die 526, a first extrusion device (e.g., a first extruder 514) is configured to form first layer 118a, and a second extrusion device (e.g., a second extruder 514) configured to form second layer 122a. The multilayer sheet may advantageously be designed and constructed such that relative ratio of layer thicknesses are selected to improve array formation processing (e.g., speed, robustness of the process, release from a mold, and/or accuracy of geometric replication) and properties of microneedle 140 (e.g., mechanical, chemical, and/or optical properties). Multilayer sheet (e.g., 110, 110a) produced by system 510 may be embossed to form array 114, as described at least with reference to FIGS. 6B and 7, such that the layer of PMMA corresponds to side 152 that defines microneedles 140 (e.g., microneedles 140 include PMMA).

Referring to FIGS. 6A-6B, FIG. 6A depicts a cross-sectional view of an example of a set of molds 610 for forming microneedle array 114, 114a-1 14d, and FIG. 6B depicts stages of an embossing process 642 to form array 114, 114a-1 14d using set of molds 610. As used herein, a mold may also be referred to as a tool. The patterns of a given embossing mold may be mated or non-mated. In a mated embossing mold, the pattern on one portion of the mold may identically, or similarly, compliment, or "mate", with the pattern on a second or other of the mated rolls. The pattern on a non-mated embossing mold does not match identically with the pattern on the other roll. Depending on the desired results, either type of embossing mold can be used. Various types of embossing processes may be useful in the formation of a microneedle array according to the methods described herein. For example, set of molds 610 may be configured to be used in an embossing process, such as plate-to-plate, roll-to-plate, or roll-to-roll hot embossing process.

Referring to FIG. 6A, set of molds 610 includes a first mold (M1) 614 and a second mold (M2) 616 that is configured to be positioned with respect to first mold (M1) 614. First mold (M1) 614 includes a proximate surface 622 that corresponds to first side 152 of microneedle array 114. First mold (M1) defines multiple cavities 626 that correspond to microneedles 140. Each of the cavities extends from a corresponding base 630 (e.g., opening) at proximal surface 622 to a distal end 636 within first mold (M1) 614 (e.g., a first tool) to define a negative mold of a microneedle. Base 630 of each of the cavities 626 has a cross-sectional area larger than that of the respective distal end 636. In a particular implementation, a cross-sectional shape of base 630 of each of the cavities 626 includes a circle. In other implementations, the cross-sectional shape of base 630 includes a shape other than a circle.

Machining to form the plurality of recesses of first mold 614 (e.g., a counter pressure element in an embossing process), second mold 616, or both, may be formed via mechanical structuring, via micro electro discharge machining, via laser percussion drilling, or according to LIGA structuring, for example. LIGA may refer to a process combining lithography, electroplating, and replication to form microstructures in steel, for example.

Second mold (M2) 616 includes a distal surface 640 and is configured to be positioned with respect to first mold (M1) 614 to define a space first mold 614 and second mold 616. In some implementations, second mold 616 may correspond to a pressure element in an embossing process that applies pressure to multi-layer sheet 110 toward (e.g., into) first mold 614. Although each of proximate surface 622 and distal surface 640 are illustrated as planar, in other implementations, proximate surface 622 and/or distal surface 640 may have a curved surface. For example, each of proximate surface 622 and distal surface 640 may have a convex surface. In a particular implementation, each of first mold (M1) 614 and second mold (M2) 616 is incorporated into a surface of a corresponding cylinder that rotates about an axis for use in a roll embossing process to form microneedle arrays. In another particular implementation, first mold (M1) 614 and/or second mold (M2) 616 are incorporated into a corresponding surface of a belt, such as a metal belt.

Referring to FIG. 6B, cross-sectional views of stages of an embossing process 642 (e.g., plate-to-plate hot embossing) using a set of molds (e.g., 610) is shown. Set of molds includes mold 646 (e.g., a counter pressure element) and, optionally, another mold (e.g., a pressure element), which has been omitted for ease of illustration. Mold 646 is associated with, or otherwise corresponds to, first mold (M1) 614 of FIG. 6A and the other mold (e.g., the pressure element) is associated with, or otherwise corresponds to, second mold (M2) 616.

At a first stage 650, multilayer sheet 110 (represented by a single crosshatching) is positioned with respect to mold 646, such that it is between mold 646 and the other mold (not shown). Multilayer sheet 110 is orientated such that first side 152 including PMMA is facing mold 646. Heat may be applied to multilayer sheet 110, such as via second side 156 by the other mold. In some implementations, one or more protective skin layers coupled to multilayer sheet 110 have been removed prior to positioning multilayer sheet as shown at first stage 650. For example, removal of the one or more protective skin layers may be achieved by physical removal (e.g., by a person or a machine).

At second stage 654, multilayer sheet 110 is brought into contact with mold 646. For example, the other mold (not shown) may apply a force to multilayer sheet 110 via second side 156 such that first side 156 contacts and is transformed (e.g., deformed) by mold 646. During the second stage 654, mold 646 and/or the other mold may be configured to heat at least a portion of multilayer sheet 110.

At a third stage 658, multilayer sheet 110 has been pressed farther into mold 646 as compared to second stage 654. At a fourth stage 662, multilayer sheet 110 has been fully pressed toward mold 646 such that first side 152 has been transformed to conform to a texture or pattern (e.g., an inverse geometry suitable for formation of microneedle array 114) of mold 646. After fourth stage 662, multilayer sheet 110 (e.g., microneedle array 114) may be disengaged from the set of molds. In some implementations, multilayer sheet 110 (e.g., microneedle array 114) may be cooled prior to and/or subsequent to disengagement from one or more molds of the set of molds.

Thus, FIG. 6B depicts an example of a hot embossing process according to aspects of the present disclosure. To illustrate, multilayer sheet 110 may be formed, and in some aspects transformed, so that an uppermost layer (e.g., first layer 118) of multilayer sheet 110 includes PMMA. The uppermost layer of multilayer sheet 110 may be situated to be the portion of the sheet 110 that initially contacts a portion of a microneedle mold (e.g., 614, 646) that is configured to form the apex or tip of microneedle 140, such that PMMA is disposed at the apex of the microneedle array mold. Accordingly, the hot embossing processes may be applied to the multilayer sheet 110 to provide production of microneedle array 114 having one or more beneficial features (e.g., a tip of PMMA) as described with reference to at least FIG. 1B.

Referring to FIG. 7, FIG. 7 shows a schematic view of an example of a system 700 for manufacturing a microneedle array from one or more polymer sheets. For example, system 700 may be configured for use in an embossing process to form microneedle array 114.

System 700 includes an apparatus 710 having a frame 714 configured to support one or more components of system 700. System 700 includes spindles 718 and pulleys 722 rotatably coupled to frame 714.

A dispenser roll 726 is coupled to frame 714 by a corresponding spindle 718 (e.g., a dispenser spindle) and includes multilayer sheet 110, such as a sheet of softened or molten multilayer polymer film. Sheet 110 includes first side 152 and second side 156. Dispenser roll 726 is configured to rotate about the first spindle to provide sheet 110 in a direction associated with arrow 730.

A tool 734 (e.g., a belt) is coupled to a first cylinder 738 and a second cylinder 742. Tool 734 is associated with a negative image, similar to first mold (M1) 614, of a microneedle shape. For example, tool 734 has a surface 760 that defines a plurality of cavities. Each of the cavities extends from a base at surface 760 to a distal end within tool 734 to define a negative mold of a microneedle (e.g., 140). The base of each of the cavities has a cross-sectional area larger than that of the respective distal end. Each of cylinders 738, 742 is configured to rotate about a corresponding rotational axis 746 coupled to frame 714. Each cylinder 738, 742 is configured to rotate bout its axis 746 in a direction indicated by arrow 750. Rotation of cylinders 738, 742 causes tool 734 to move about cylinders 738, 742 in a direction indicated by arrow 754. In some implementations, first cylinder 738 is heated and is configured to heat at least a portion of tool 734 that is in contact with first cylinder. Additionally, or alternatively, second cylinder 742 is cooled and is configured to cool at least a portion of tool 734 that is in contact with second cylinder.

One or more rollers 770 are coupled to frame 714 and disposed at a corresponding angular position relative to rotational axis 746 of first cylinder 738. For example, roller 770a is disposed at a first angular position relative to rotational axis 746 of cylinder 738 and roller 770b is disposed at a second angular position relative to rotational axis 746 of cylinder 738. Each of the one or more rollers 770 is configured to press sheet 110 against tool 734 such that a material (e.g., PMMA) of first side 152 enters into openings (e.g., cavities) of tool 734 to define a plurality of microneedles (e.g., 140). As referred to herein, a portion of sheet 110 having microneedles 140 formed thereon is referred as a sheet 778.

A receiver roll 774 is coupled to frame 714 by a corresponding spindle 718 (e.g., a receiver spindle) and is configured to receive sheet 778 after definition of the microneedles 140 on first side 152 of the multilayer polymer. Sheet 778 includes one or more portions 780 that each includes a microneedle array (e.g., microneedle array 114) that includes microneedles 140.

It is noted that in some implementations, multilayer sheet 110 stored on dispenser roll 726 includes one or more skin layers as previously described herein. At least one of the one or more skin layers may be removed prior to sheet 110 being provided to tool 734. For example, a protective skin in contact with side 152 may be removed to expose side 152 (e.g., surface) prior to side 152 contacting tool 734. The one or more protective skin layers may include polypropylene or polycarbonate, as illustrative examples. It is also noted that in some implementations, dispenser roll 726 can be omitted and multilayer sheet 110 can be provided to tool 734 from an extrusion system, such as system 510.

During operation of system 700, sheet 110 is provided from dispenser roll 726 to tool 734. Sheet 110 is provided between tool 734 (e.g., first cylinder 738) and one or more rollers 770, and pressed into the negative mold of tool 734 to form microneedles. In a particular implementation, one or more rollers 770, a portion of tool 734, cylinder 738, or a combination thereof, may be at a temperature sufficient to heat sheet 110 above the sheet's glass transition temperature. After being pressed between one or more rollers 770 and tool 734, the pressed sheet 778 is provided toward cylinder 738, in a direction indicated by arrow 784, and the pressed sheet reaches a temperature zone (e.g., a lower temperature) and solidifies or hardens as sheet 778. Sheet 778 is provided to receiver roll 774 as indicated by arrow 782. In some implementations, receiver roll 774 is omitted and sheet 778 is provided for further processing, such as further processing to divide sheet 778 into individual microneedle arrays.

Thus, FIG. 7 depicts system 700 configured to manufacture microneedle array 114. System 700 enable arrays (e.g., large arrays) of microneedles to be manufactured more rapidly than prior art methods. Additionally, system 700 can produce one or more arrays extending along an elongated sheet rather than being limited to producing one singular discrete array at a time. For example, a sheet with a length that is five or more times its width can have one or more microneedle arrays extending along a majority of its length. The ability to produce multiple microneedle arrays reduces time and cost of manufacturing microneedle arrays.

Referring to FIG. 8, method 800 may be performed by a manufacturing device or system, such as system 700. The microneedle array includes a plurality of microneedles and may include or correspond to microneedle array 114, 114a-1 14d, as described herein.

Method 800 includes disposing a multilayer polymer between a pressing device and a surface of the tool, at 810. A temperature of at least a portion of the multilayer polymer in contact with the tool is above the multilayer polymer's glass transition temperature. The multilayer polymer (e.g., one or more sheets of polymer) includes a first layer and a second layer; the first layer including polymethylmethacrylate (PMMA) and the second layer including that includes polycarbonate (PC). For example, the multilayer polymer may include or correspond to sheet 110, 110a, or a combination thereof, and first and second layers can be associated with or correspond to first layer 118, 118a, 144, and second layer 122, 122a, 148, respectively. The tool may include or correspond to first mold (M1) 614, mold 646, tool 734, or a combination thereof. The pressing device may include or correspond to second mold (M2) 616, one or more of rollers 770, or a combination thereof. In some implementations, the pressing device includes another tool and the polymer is in a molten state as it is disposed between the tool and the pressing device (i.e., the other tool).

In a particular implementation, the first layer (including PMMA) has a first glass transition temperature and the second layer (including PC) has a second glass transition temperature. The glass transition temperature of the multilayer polymer can be the lower of the first glass transition temperature and the second glass transition temperature.

Method 800 also includes pressurizing at least a portion of the multilayer polymer between the pressing device and the tool, at 812. At least the portion of the multilayer polymer may be pressurized such that the multilayer polymer defines a layer of polymer between the pressing device and the surface of the tool (the multilayer polymer having a first side of the first layer facing the tool and a second side of the second layer facing the pressing device), and such that the polymer enterse into cavities of the tool until the polymer substantially fills the cavities to form a plurality of microneedles on the first side. In some implementations, the plurality of microneedles are substantially solid. The first side of the layer and the second side of the layer may be associated with or correspond to first surface/side 126, 152 and second surface/side 130, 156, respectively.

In some implementations, method 800 may also include forming the multilayer polymer (e.g., 110). Forming the multilayer polymer can include receiving a first material comprising the PMMA and receiving a second material comprising the PC, forming the first layer and forming the second layer; and coupling the first layer and the second layer to form the multilayer polymer. To illustrate, in a particular implementation, the first layer is formed by a first extrusion process and the second layer is formed by a second extrusion process. Forming of the multilayer polymer may be performed by system 510 of FIG. 5.

One product or article of manufacture that can be formed from method 800 includes a multilayer polymer having a first side and an opposing second side, the multilayer polymer defining a plurality of microneedles on the first side of the layer, each microneedle having a distal end and a base between the distal end and the second side of the multilayer polymer, the base of each of the microneedles having a cross-sectional area larger than that of the respective distal end. The multilayer polymer includes a first layer having the first side and that includes polymethylmethacrylate (PMMA), and includes a second layer having the second side and that includes polycarbonate (PC).

Thus, method 800 describes manufacturing of an array of microneedles, such as microneedle array 114, 114a-114d. Method 800 advantageously enable arrays (e.g., large arrays) of microneedles, and multiple arrays of microneedles, to be manufactured more rapidly than prior art methods and at a lower cost. Additionally, microneedle arrays manufactured according to method 800 can beneficially have aspect ratios for microneedles with sharp tips (of PMMA) to penetrate dermal surfaces (while still maintaining the benefit of being relatively pain free) and to maintain their structural integrity and strength during use. For example, the combination of PMMA and PC in the microneedle array obtains the hardness of PMMA (especially in the tip area) and incorporates the toughness behavior of PC to form the microneedle array. The microneedle arrays may also have desired varying aspect ratio, strength, and mechanical performance sufficient to provide a sharp tip among the microneedles and a sharp blade to properly penetrate or cut the skin.

The claims are not intended to include, and should not be interpreted to include, means-plus- or step-plus-function limitations, unless such a limitation is explicitly recited in a given claim using the phrase(s) "means for" or "step for," respectively.

## Claims

1. A microneedle array (114) comprising:
a multilayer polymer having a first side (126, 152) and an opposing second side (130, 156), the multilayer polymer defining a plurality of microneedles (140) on the first side (126, 152), each microneedle (140) having a distal end (164) and a base (160) between the distal end (164) and the second side (130, 156) of the multilayer polymer, the base (160) of each of the microneedles (140) having a cross-sectional area larger than that of the respective distal end (164);
where the multilayer polymer comprises:
a first layer (118, 144) having the first side (126, 152) and that includes polymethylmethacrylate (PMMA); and
a second layer (122, 148) having the second side (130, 156) and that includes polycarbonate (PC)
wherein a hardness of the first layer (118, 144) comprises a pencil hardness of H or greater.

2. The microneedle array (114) of claim 1, where the multilayer polymer includes fewer than three layers.

3. The microneedle array (114) of any of claims 1-2, where the first layer (118, 144) is in contact with the second layer (122, 148).

4. The microneedle array (114) of any of claims 1-3, where the multilayer polymer further comprises at least a third layer (410) positioned between the first layer (118, 144) and the second layer (122, 148).

5. The microneedle array (114) of claim 4, where the third layer (410) includes polyester or a resin, or where the third layer (410) includes a polymer selected from the group consisting of polycarbonate (PC), polymethylmethacrylate (PMMA), liquid-crystal polymer (LCP), polyether ether ketone (PEEK), fluorinated ethylene propylene (FEP), polysulfone (PSU), polyethylenimine, polyetherimide, polyimide (PI), polycarbonate copolymer (PC COPO), cyclic olefin copolymer (COC), cyclo olefin polymer (COP), polyamide (PA), acrylonitrile butadiene styrene (ABS), and polyphenylene ether (PPE).

6. The microneedle array (114) of any of claims 1-5, where a surface of the first layer (118, 144) that corresponds to the first side (126, 152) defines an exposed surface of the plurality of microneedles (140).

7. The microneedle array (114) of any of claims 1-6, where the first layer (118, 144) comprises a cross-linked material that includes the PMMA.

8. The microneedle array (114) of any of claims 1-5 and 7, further comprising a hardcoat layer coupled to the first side (126, 152) of the multilayer polymer.

9. A method of manufacturing a microneedle array (114), the method comprising:
disposing a multilayer polymer between a pressing device and a surface (622, 760) of a tool (614, 734) with a temperature of at least a portion of the multilayer polymer in contact with the tool (614, 734) above a glass transition temperature of the multilayer polymer, the tool (614, 734) defining a plurality of cavities (626), each of the cavities (626) extending from a base (630) at the surface (622, 760) to a distal end (636) within the tool (614, 734) to define a negative mold of a microneedle (140), the base (630) of each of the cavities (626) having a cross-sectional area larger than that of the respective distal end (636); and
pressurizing at least a portion of the multilayer polymer between the pressing device and the tool (614, 734) such that the multilayer polymer:
defines a layer of polymer between the pressing device and the surface (622, 760) of the tool (614, 734), the multilayer polymer having a first side (126, 152) facing the tool (614, 734) and a second side (130, 156) facing the pressing device; and
flows into the cavities (626) until the polymer substantially fills the cavities (626) to form a plurality of microneedles (140) on the first side (126, 152);
where the multilayer polymer comprises:
a first layer (118, 144) having the first side (126, 152) and that includes polymethylmethacrylate (PMMA); and
a second layer (122, 148) having the second side (130, 156) and that includes polycarbonate (PC)
wherein a hardness of the first layer (118, 144) comprises a pencil hardness of H or greater.

10. The method of claim 9, where the first layer (118, 144) has a first glass transition temperature, the second layer (122, 148) has a second glass transition temperature, and the glass transition temperature of the multilayer polymer comprises the lower of the first glass transition temperature and the second glass transition temperature.

11. The method any of claims 9-10, further comprising forming the multilayer polymer.

12. The method of claims 11, where forming the multilayer polymer comprises:
receiving a first material (540) comprising the PMMA;
forming the first layer (118, 144);
receiving a second material (542) comprising the PC;
forming the second layer (122, 148); and
coupling the first layer (118, 144) and the second layer (122, 148) to form the multilayer polymer.

13. The method of claims 9-12, where the first layer (118, 144) is formed by a first extrusion process and the second layer (122, 148) is formed by a second extrusion process..

## Patentansprüche

1. Mikronadelanordnung (114), die Folgendes umfasst:
ein mehrschichtiges Polymer mit einer ersten Seite (126, 152) und einer gegenüberliegenden zweiten Seite (130, 156), wobei das mehrschichtige Polymer eine Vielzahl von Mikronadeln (140) auf der ersten Seite (126, 152) definiert, wobei jede Mikronadel (140) ein distales Ende (164) und eine Basis (160) zwischen dem distalen Ende (164) und der zweiten Seite (130, 156) des mehrschichtigen Polymers aufweist, wobei die Basis (160) jeder der Mikronadeln (140) eine größere Querschnittsfläche als die des jeweiligen distalen Endes (164) aufweist;
wobei das mehrschichtige Polymer Folgendes umfasst:
eine erste Schicht (118, 144) mit der ersten Seite (126, 152), die Polymethylmethacrylat (PMMA) enthält; und
eine zweite Schicht (122, 148) mit der zweiten Seite (130, 156), die Polycarbonat (PC) enthält
wobei eine Härte der ersten Schicht (118, 144) eine Bleistifthärte von H oder größer aufweist.

2. Mikronadelanordnung (114) nach Anspruch 1, wobei das mehrschichtige Polymer weniger als drei Schichten umfasst.

3. Mikronadelanordnung (114) nach einem der Ansprüche 1 bis 2, wobei die erste Schicht (118, 144) in Kontakt mit der zweiten Schicht (122, 148) steht.

4. Mikronadelanordnung (114) nach einem der Ansprüche 1 bis 3, wobei das mehrschichtige Polymer ferner mindestens eine dritte Schicht (410) umfasst, die zwischen der ersten Schicht (118, 144) und der zweiten Schicht (122, 148) angeordnet ist.

5. Mikronadelanordnung (114) nach Anspruch 4, wobei die dritte Schicht (410) Polyester oder ein Harz enthält, oder wobei die dritte Schicht (410) ein Polymer enthält, das ausgewählt ist aus der Gruppe bestehend aus Polycarbonat (PC), Polymethylmethacrylat (PMMA), flüssigkristallinem Polymer (Liquid-Crystal Polymer, LCP), Polyetheretherketon (PEEK), fluoriertem Ethylenpropylen (FEP), Polysulfon (PSU), Polyethylenimin, Polyetherimid, Polyimid (PI), Polycarbonat-Copolymer (PC COPO), zyklischem Olefin-Copolymer (COC), Cyclo-Olefin-Polymer (COP), Polyamid (PA), AcrylnitrilButadien-Styrol (ABS) und Polyphenylenether (PPE).

6. Mikronadelanordnung (114) nach einem der Ansprüche 1 bis 5, wobei eine Oberfläche der ersten Schicht (118, 144), die der ersten Seite (126, 152) entspricht, eine freiliegende Oberfläche der Vielzahl von Mikronadeln (140) definiert.

7. Mikronadelanordnung (114) nach einem der Ansprüche 1 bis 6, wobei die erste Schicht (118, 144) ein vernetztes Material umfasst, das das PMMA enthält.

8. Mikronadelanordnung (114) nach einem der Ansprüche 1 bis 5 und 7, die ferner eine Hartbeschichtungsschicht umfasst, die mit der ersten Seite (126, 152) des mehrschichtigen Polymers verbunden ist.

9. Verfahren zur Herstellung einer Mikronadelanordnung (114), wobei das Verfahren Folgendes umfasst:
Anordnen eines mehrschichtigen Polymers zwischen einer Pressvorrichtung und einer Oberfläche (622, 760) eines Werkzeugs (614, 734), wobei eine Temperatur von mindestens einem Teil des mehrschichtigen Polymers, der in Kontakt mit dem Werkzeug (614, 734) ist, über einer Glasübergangstemperatur des mehrschichtigen Polymers liegt, wobei das Werkzeug (614, 734) eine Vielzahl von Hohlräumen (626) definiert, wobei sich jeder der Hohlräume (626) von einer Basis (630) an der Oberfläche (622, 760) zu einem distalen Ende (636) innerhalb des Werkzeugs (614, 734) erstreckt, um eine negative Form einer Mikronadel (140) zu definieren, wobei die Basis (630) jedes der Hohlräume (626) eine größere Querschnittsfläche als die des jeweiligen distalen Endes (636) aufweist; und
Druckbeaufschlagung mindestens eines Teils des mehrschichtigen Polymers zwischen der Pressvorrichtung und dem Werkzeug (614, 734), so dass das mehrschichtige Polymer:
eine Schicht aus Polymer zwischen der Pressvorrichtung und der Oberfläche (622, 760) des Werkzeugs (614, 734) definiert, wobei das mehrschichtige Polymer eine erste Seite (126, 152), die dem Werkzeug (614, 734) zugewandt ist, und eine zweite Seite (130, 156), die der Pressvorrichtung zugewandt ist, aufweist; und
in die Hohlräume (626) fließt, bis das Polymer die Hohlräume (626) im Wesentlichen füllt, um eine Vielzahl von Mikronadeln (140) auf der ersten Seite (126, 152) zu bilden;
wobei das mehrschichtige Polymer Folgendes umfasst:
eine erste Schicht (118, 144) mit der ersten Seite (126, 152), die Polymethylmethacrylat (PMMA) enthält; und
eine zweite Schicht (122, 148) mit der zweiten Seite (130, 156), die Polycarbonat (PC) enthält
wobei eine Härte der ersten Schicht (118, 144) eine Bleistifthärte von H oder größer aufweist.

10. Verfahren nach Anspruch 9, wobei die erste Schicht (118, 144) eine erste Glasübergangstemperatur aufweist, die zweite Schicht (122, 148) eine zweite Glasübergangstemperatur aufweist, und die Glasübergangstemperatur des mehrschichtigen Polymers die niedrigere der ersten Glasübergangstemperatur und der zweiten Glasübergangstemperatur umfasst.

11. Verfahren nach einem der Ansprüche 9 bis 10, das ferner das Bilden des mehrschichtigen Polymers umfasst.

12. Verfahren nach Anspruch 11, wobei das Bilden des mehrschichtigen Polymers Folgendes umfasst:
Aufnehmen eines ersten Materials (540), das das PMMA umfasst;
Bilden der ersten Schicht (118, 144);
Aufnehmen eines zweiten Materials (542), das das PC umfasst; Bilden der zweiten Schicht (122, 148); und
Verbinden der ersten Schicht (118, 144) und der zweiten Schicht (122, 148) zur Bildung des mehrschichtigen Polymers.

13. Verfahren nach Anspruch 9 bis 12, wobei die erste Schicht (118, 144) durch ein erstes Extrusionsverfahren und die zweite Schicht (122, 148) durch ein zweites Extrusionsverfahren gebildet wird.

## Revendications

1. Réseau de micro-aiguilles (114) comprenant :
un polymère multicouche ayant un premier côté (126, 152) et un deuxième côté opposé (130, 156), le polymère multicouche définissant une pluralité de micro-aiguilles (140) sur le premier côté (126, 152), chaque micro-aiguille (140) ayant une extrémité distale (164) et une base (160) entre l'extrémité distale (164) et le deuxième côté (130, 156) du polymère multicouche, la base (160) de chacune des micro-aiguilles ( 140) ayant une section transversale plus grande que celle de l'extrémité distale respective (164) ;
où le polymère multicouche comprend :
une première couche (118, 144) ayant le premier côté (126, 152) et qui comprend du polyméthylméthacrylate (PMMA) ; et
une deuxième couche (122, 148) ayant le deuxième côté (130, 156) et qui comprend du polycarbonate (PC)
dans lequel une dureté de la première couche (118, 144) comprend une dureté au crayon de H ou plus.

2. Réseau de micro-aiguilles (114) selon la revendication 1, dans lequel le polymère multicouche comprend moins de trois couches.

3. Réseau de micro-aiguilles (114) selon l'une quelconque des revendications 1 à 2, où la première couche (118, 144) est en contact avec la deuxième couche (122, 148).

4. Réseau de micro-aiguilles (114) selon l'une quelconque des revendications 1 à 3, où le polymère multicouche comprend en outre au moins une troisième couche (410) positionnée entre la première couche (118, 144) et la deuxième couche (122, 148).

5. Réseau de micro-aiguilles (114) selon la revendication 4, où la troisième couche (410) comprend du polyester ou une résine, ou où la troisième couche (410) comprend un polymère choisi dans le groupe constitué de polycarbonate (PC), polyméthylméthacrylate (PMMA), polymère à cristaux liquides (LCP), polyéther éther cétone (PEEK), éthylène propylène fluoré (FEP), polysulfone (PSU), polyéthylèneimine, polyétherimide, polyimide (PI), copolymère de polycarbonate (PC COPO), copolymère de cyclooléfine (COC), polymère de cyclooléfine (COP), polyamide (PA), acrylonitrile butadiène styrène (ABS) et polyphénylène éther (PPE).

6. Réseau de micro-aiguilles (114) selon l'une quelconque des revendications 1 à 5, où une surface de la première couche (118, 144) qui correspond au premier côté (126, 152) définit une surface exposée de la pluralité de micro-aiguilles (140).

7. Réseau de micro-aiguilles (114) selon l'une quelconque des revendications 1 à 6, où la première couche (118, 144) comprend un matériau réticulé qui comprend le PMMA.

8. Réseau de micro-aiguilles (114) selon l'une quelconque des revendications 1 à 5 et 7, comprenant en outre une couche de revêtement dur couplée au premier côté (126, 152) du polymère multicouche.

9. Procédé de fabrication d'un réseau de micro-aiguilles (114), le procédé comprenant :
la disposition d'un polymère multicouche entre un dispositif de pression et une surface (622, 760) d'un outil (614, 734) avec une température d'au moins une partie du polymère multicouche en contact avec l'outil (614, 734) au-dessus d'une température de transition vitreuse du polymère multicouche, l'outil (614, 734) définissant une pluralité de cavités (626), chacune des cavités (626) s'étendant à partir d'une base (630) au niveau de la surface (622, 760) à une extrémité distale (636) à l'intérieur de l'outil (614, 734) pour définir un moule négatif d'une micro-aiguille (140), la base (630) de chacune des cavités (626) ayant une surface de section transversale plus grande que celle de l'extrémité distale respective (636) ; et
la pressurisation d'au moins une partie du polymère multicouche entre le dispositif de pression et l'outil (614, 734) de sorte que le polymère multicouche :
définit une couche de polymère entre le dispositif de pression et la surface (622, 760) de l'outil (614, 734), le polymère multicouche ayant une première face (126, 152) tournée vers l'outil (614, 734) et une deuxième face (130, 156) tournée vers le dispositif de pression ; et
s'écoule dans les cavités (626) jusqu'à ce que le polymère remplisse sensiblement les cavités (626) pour former une pluralité de microaiguilles (140) sur le premier côté (126, 152) ;
où le polymère multicouche comprend :
une première couche (118, 144) ayant le premier côté (126, 152) et qui comprend du polyméthylméthacrylate (PMMA) ; et
une deuxième couche (122, 148) ayant le deuxième côté (130, 156) et qui comprend du polycarbonate (PC)
dans lequel une dureté de la première couche (118, 144) comprend une dureté au crayon de H ou plus.

10. Procédé selon la revendication 9, dans lequel la première couche (118, 144) a une première température de transition vitreuse, la deuxième couche (122, 148) a une deuxième température de transition vitreuse, et la température de transition vitreuse du polymère multicouche comprend la plus faible de la première température de transition vitreuse et la deuxième température de transition vitreuse.

11. Procédé selon l'une quelconque des revendications 9 à 10, comprenant en outre la formation du polymère multicouche.

12. Procédé selon la revendication 11, dans lequel la formation du polymère multicouche comprend :
la réception d'un premier matériau (540) comprenant le PMMA ;
la formation de la première couche (118, 144) ;
la réception d'un deuxième matériau (542) comprenant le PC ;
la formation de la deuxième couche (122, 148) ; et
le couplage de la première couche (118, 144) et de la deuxième couche (122, 148) pour former le polymère multicouche.

13. Procédé selon les revendications 9 à 12, dans lequel la première couche (118, 144) est formée par un premier procédé d'extrusion et la deuxième couche (122, 148) est formée par un deuxième procédé d'extrusion.
